(19)

Europäisches
Patentamt
European
Patent Office
Office européen
des brevets

(11) **EP 4 268 798 A1**

(12) **EUROPEAN PATENT APPLICATION**
published in accordance with Art. 153(4) EPC

(43) Date of publication:
**01.11.2023 Bulletin 2023/44**

(21) Application number: **21908172.6**

(22) Date of filing: **22.12.2021**

(51) International Patent Classification (IPC):
*A61K 8/27* $^{(2006.01)}$           *A61K 8/29* $^{(2006.01)}$
*C07G 1/00* $^{(2011.01)}$           *A61Q 17/04* $^{(2006.01)}$

(86) International application number:
**PCT/BR2021/050574**

(87) International publication number:
**WO 2022/133566 (30.06.2022 Gazette 2022/26)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **22.12.2020  BR 102020026475**

(71) Applicant: **Botica Comercial Farmacêutica Ltda
83055-900 São José dos Pinhais (BR)**

(72) Inventors:
• **HARAGUCHI PADILHA, Milene
80330-000 Curitiba (BR)**
• **SCLIAR SASSON, Clarice
82220-170 Curitiba (BR)**
• **CORREA DA CRUZ, Janny Fernanda
83025-320 Curitiba (BR)**
• **STANGHERLIN SANTUCCI, Beatriz
3471-030 São Domingos (BR)**

(74) Representative: **Winter, Brandl - Partnerschaft
mbB
Alois-Steinecker-Straße 22
85354 Freising (DE)**

(54) **METHOD FOR OBTAINING A LIGNIN COMPOSITION ASSOCIATED WITH ZNO AND T1O2 FOR A COSMETIC PRODUCT WITH COLORING FOR BLACK SKIN, TO BOOST BLUE LIGHT PROTECTION, AND TO PROTECT THE SKIN AGAINST EXTERNAL OXIDIZING AGENTS, AND RESPECTIVE OBTAINED PRODUCT**

(57)     The present invention relates to the processing and product obtained from lignin, and therefore pertains to the technical field of "management and treatment of waste products/reuse of used materials", and more specifically to the "recovery or reuse of waste products". The present patent of invention also pertains to the technical field of cosmetics, preparations for makeup and natural organic macromolecular compounds derived from lignocellulosic materials, and also the preparation or chemical processing thereof. More specifically, the invention relates to the process for obtaining a lignin composition associated with zinc oxide (ZnO) and titanium dioxide (TiOz) for cosmetic products with coloring for black skin, to boost blue light protection, and to protect the skin against external oxidizing agents, and the respective obtained product, in which at least 0.5%, preferably 0.76%, of the raw material of said product is made up of said nanostructured lignin.

EP 4 268 798 A1

**Description**

The present invention relates to the processing and product obtained from lignin, and therefore pertains to the technical field of "management and treatment of waste products/reuse of used materials", and more specifically to the "recovery or reuse of waste products". The present patent of invention also pertains to the technical field of cosmetics, preparations for makeup and natural organic macromolecular compounds derived from lignocellulosic materials, and also the preparation or chemical processing thereof. More specifically, it refers to the process for obtaining a lignin composition associated with Zinc Oxide (ZnO) and Titanium Dioxide ($TiO_2$) for cosmetic products with coloring for black skin, to boost blue light protection, and to protect the skin against external oxidizing agents, and respective resulting product.

**STATE OF THE ART**

Various documents from the state of the art already refer to the use of lignin and/or the association of ZnO and $TiO_2$ in the technical field of cosmetics applied to skin care, and they also mention their beneficial characteristics, such as protection against ultraviolet radiation (UV), or even in obtaining new color tones.

For example, patent document CN106309212A presents a skin care cosmetic capable of resisting ultraviolet radiation and a preparation method thereof, wherein said cosmetic comprises at least lignin, water, a chrysanthemum extract, xylitol, propanodiol, polyethylene glycol, EDTA (ethylenediaminetetracetic acid disodium), citric acid, sodium hyaluronate, glycerin, allantoin, iodopropynyl butylcarbamate and essence. The cosmetic has the function of resisting ultraviolet radiation, lignin being adopted due to its high ultraviolet absorption capacity.

Patent document JP2006052197 is designed to obtain a cosmetic that besides having the property of retaining moisture and antioxidant activity, has a differentiated color tone, and said cosmetic comprises water-soluble lignin applied to various cosmetics in various ways. The cosmetic is presented in the form of a powder, having a brown to reddish purple color tone, more specifically, it changes from a yellowish-reddish brown to a reddish purple color.

In turn, patent document US6500411, describes cosmetic and personal grooming compositions using particulate UVR filters, such as $TiO_2$ and ZnO. Another important component of the multifunctional additive composition is a dispersant or surface modifier for the previous particulate materials, selected from the family of polyphenol natural polymers, such as lignosulphonates, lignins, humates, tannates and derivatives thereof.

Another sun protection composition is presented by patent document KR20190109032, comprising from 10 to 30 parts by weight of lignin or modified lignin, based on 100 parts by weight of the total composition. The sun filter composition comprises at least an additive in an amount from 0.1 to 2 parts by weight, and a cosmetic composition comprising lignin or modified lignin within the ranges above, while it solves problems such as skin toxicity due to conventional sun filter compounds. In one embodiment, in the composition with sun block effect of document KR20190109032, the inorganic sun filter is comprised by $TiO_2$, ZnO and iron oxides.

Patent document KR20190083182 presents a lignin-base cosmetic sun filter composition, wherein the physical blocker reflects and disperses the ultraviolet rays and prevents penetration into the skin, and is classified as an inorganic sun filter (ultraviolet dispersion agent). Examples thereof include $TiO_2$ and ZnO.

Although $TiO_2$ is commonly used as sun filter, patent document WO2017197530 seeks to approach concerns regarding the catalytic activity of $TiO_2$ and its generation of oxygen-reactive species, providing nanoparticles of $TiO_2$ coated with lignin, producing a fine-coated metal oxide nanoparticle. Other lignin-coated metal oxides are also disclosed, as are methods for preparing lignin-coated metal oxide nanoparticles. Said document WO2017197530 also mentions that the fine lignin coating does indeed make these compositions cosmetically acceptable, not only in terms of absorption and light dispersion, but also in terms of visible color and appearance, wherein the lignin and the TiOz provide a very light tone, suitable for skin care formulations.

Similarly, patent document WO2005048970 also presents dermatological and cosmetic preparations with at least a lignin, and may also comprise inorganic pigments commonly used in cosmetics for protecting the sking against ultraviolet rays, with particular preference given to TiOz-based pigments.

Another sun filter composition containing in its aqueous/liquid phase an additive capable of increasing the classification of the sun protection factor by at least 20% is presented by patent document WO2010093573. Preferred interfacially-active polymers are presented, including lignin, and preferred particulate materials, including $TiO_2$ and ZnO.

Further, patent document WO2017093184 presents a study of the effect of the presence of different nanoparticles (TiOz and ZnO) in the rheological properties of the lignin adsorption layer.

Among documents from non-patent literature, José Manuel Gutiérrez-Hernández et al, in his article entitled "Use of Agave Tequilana-Lignin and Zinc Oxide Nanoparticles for Skin Photoprotection" (Journal of photochemistry and photobiology. B, Biology, October, 2016), describes the importance of using sun protectors for preventing damage to the skin in human beings, emphasizing that inorganic active components, such as zinc oxide (ZnO), have been commonly used in sun filters due to their capacity to block UV radiation. This UV protection can be improved to cover UV protection bands when combined with other components, such as titanium dioxide (TiO$_2$). Said publication evaluates the photoprotection properties of organic nanoparticles made from lignin in combination with nanoparticles of ZnO as active ingredients for sun filters, and highlights that lignin nanoparticles have the additional advantage of being of an organic nature and its brown color can be used to combine with the skin tone of the person using it. Therefore, the whitish tone caused by sun protectors with ZnO can be avoided and a correspondence can also be made with the skin tone to adapt the sun filter to a specific person. José Manuel Gutiérrez-Hernández *et al,* therefore, indicates that this biopolymer is an excellent candidate to be considered as an active component in cosmetics that protect the skin, with the advantage that it also provides a pleasant tone to the skin and not the typical whitish tone, which is unpleasant for people with darker skin tones. Lastly, it recognizes that although the formulation of lignin still requires optimization, the results shown in his Article suggest a new option of sustainable resources for use in photoprotection.

Lastly, in addition to the documents referred to above, publications in documents and patent, such as in IN201811048498, JP3751630 and WO18004194, highlight the antioxidant activity characteristic present in compositions comprising lignin, and that additionally present the use of DPPH tests/assays to prove this inherent oxidant property. Moreover, at least document IN201811048498, besides emphasizing the antioxidant capacity, associates the use of lignin nanocomposites with ZnO and TiO$_2$.

**TECHNICAL PROBLEM**

As demonstrated, it is possible to note that the lignin composition with ZnO and TiO$_2$, especially in the formulation of sun filters, is already known in the state of the art, since the study and application thereof occurs on a recurrent basis.

Nevertheless, as highlighted by José Manuel Gutiérrez-Hernández *et al,* the need remains to optimize and develop the lignin formulation as a resource for photoprotection and as an active component in cosmetics.

With regards to the toning effect of the use of lignin in the mixture as cosmetic, the state of the art which associates the use of said lignin to ZnO and/or TiOz does not provide in depth details of the process steps that enable a cosmetic product to be obtained with specific coloring optimized for black skins, but merely describes, in a superficial way, the toning effect of the mixture, describing a "brown to reddish lilac" coloring and its possible application in cosmetics as sun block, moisturizers and body lotions, mascara, base, face powder, eye shadow, and others.

Moreover, finding the tone of the ideal base according to the most tones most suited to Brazilian consumers continues to be one of the main desires of the cosmetics industry. Understanding the needs of consumers in relation to this category is a major challenge for the development of these products. The market offers various structural options of facial liquid base, but the colors do not satisfy 100% of the Brazilian population, which is highly miscegenated.

Therefore, the technical problem persists of having grayish tones in cosmetic formulations with coloring for black skin due to the presence of physical filters, as these confer a white and opaque background.

Consequently, makeup that uniformly satisfies color integrity for the tones and subtones of the Brazilian population is considered attractive and innovative. A major step forward in the development of these tones versus each effect of application, by means of more assertive products, has become necessary in recent years.

Regarding the matter of protection against ultraviolet rays, it is also important to consider the hyperpigmentation caused by the action of blue light on the skin, which provokes an increase in melanin and consequent appearance of stains that are harder to remove than the stains caused by UV radiation. So it is still necessary that the use of lignin associated to ZnO and/or TiO$_2$ can be boosted so as to inhibit the increase in melanin induced by exposure to blue light.

Moreover, despite the anticipation of the antioxidant property of lignin by documents IN201811048498, JP3751630 and WO18004194 referred to above, there is no evidence of a specific concentration value of a nanostructured lignin in a skin protection product that is capable of reducing by 50% the oxidation of the DPPH (common abbreviation for the organic chemical compound 2,2-diphenyl-1-picryl-hydrazyl) assay radical and at the same time is capable of assuring, concomitantly, desirable cosmetic product characteristics with coloring for black skin and to boost blue light protection.

Therefore, in general terms, there is still a need to obtain a cosmetic that incorporates lignin as "booster" for sun protection against ultraviolet rays (UVB and UVA) and against blue light, with a pre-mixture with ZnO and TiOz and with a new cosmetic base tone geared for darker skin tones, as well as the use of lignin in specific proportions capable of protecting the skin from external oxidizing agents.

**TECHNICAL SOLUTION**

In complement to the current art and in the sense of achieving the unprecedented technical effect of a process of obtaining a composition comprising lignin associated with mixtures of a suspension of ZnO and with mixtures of a dispersion composition of TiO$_2$, by means of the present invention, the problem is solved of having grayish tones in cosmetic formulations with coloring for black skin due to the presence of physical filters that confer a white and opaque background, making this type of product and development more adaptable to any skin tone, even in the presence of high concentrations of physical filters and/or other sensorial powders that confer a white background to face makeup.

The present invention involves a special selection of particular process conditions that produces unexpected technical effects in the resulting product, additionally achieving a cosmetic product with coloring for black skin, to boost blue light protection and to protect the skin against external oxidizing agents.

## BRIEF DESCRIPTION OF THE DRAWINGS

The description below is not limited to the diagrams or images cited, the reference being the following illustrations referenced below.

Figure 1 presents an example of the technical problem of having grayish tones in usual cosmetic formulations for black skin due to the presence of physical filters.

Figure 2A presents an example of the visual appearance resulting from applying a usual cosmetic product on the top half of the face of a dark skin tone user.

Figure 2B presents an example of the visual appearance resulting from applying a cosmetic product of the present invention on the top half of the face of a dark skin tone user.

Figure 3 presents a graph with a comparative result between tones from the samples achieved usually without lignin and with lignin under the conditions of associating mixtures of ZnO and of $TiO_2$ of the present invention.

Figure 4 presents a graph of the antioxidant activity by DPPH based on the sample concentration of nanostructured lignin.

## DETAILED DESCRIPTION

The present invention pertains to a process for obtaining a lignin associated with ZnO and $TiO_2$ for cosmetic products with coloring for dark skin tones, to boost blue light protection in cosmetic formulations and to protect the skin against oxidizing agents.

Initially, the present invention adopts the use of a pre-processed dry lignin of high molecular weight obtained from cellulose fibers.

The lignin processed by the present invention undergoes a chemical extraction process by means of steps of filtering, washing and drying, obtaining a lignin with desirably high molecular weight to be adopted in association with ZnO and $TiO_2$.

The present invention uses lignin of high molecular weight, said lignin having ideal *in vitro* assay indexes in terms of phototoxicity for cosmetic application.

Once the optimized lignin is obtained, specific mixtures of a suspension of ZnO and also a dispersion composition of $TiO_2$ are added to said lignin.

Table I below lists the components used in the suspension of ZnO, as well as their characteristics of percentage by weight, according to the present invention.

| Table I | |
| --- | --- |
| Chemical component | Weight % |
| Zinc Oxide | 61.50 to 64.50 |
| Butyloctyl Salicylate | 31.00 to 35.00 |
| Triethoxyaprilylsilane | 1.00 to 5.00 |
| Alkyl Benzoate C12-C15 | 1.00 to 2.50 |

Table II below lists the components used in the dispersion composition of $TiO_2$, as well as their characteristics of percentage by weight, according to the present invention.

| Table II | |
| --- | --- |
| Chemical component | Weight % |
| Dodecamethylpentasiloxane | 0 to 60 |
| Titanium Dioxide | 20 to 30 |

(continued)

| Table II | |
| --- | --- |
| Chemical component | Weight % |
| Organo polysiloxane-modified polyglycerin | 5 to 10 |

For improved result of the cosmetic resulting from the association of the components listed above, the particles of TiOz (present in the mineral Rutile) used in the present invention, are sized up to 10 nm in width and up to 50 nm in length.

Figure 2A shows an example of the visual appearance resulting from applying a usual cosmetic product with lignin, but without said lignin having the specific composition according to the present invention, on the top half of the face of a dark skin tone user, and it is possible to perceive the difference in the resulting tone on the face of the user.

In contrast, Figure 2B shows the visual appearance resulting from applying a cosmetic product, wherein the lignin is presented in a specific composition according to the present invention, on the top half of the face of a dark skin tone user, and it is impossible to see any difference in the resulting tone on the face of the user.

Relative to the unexpected benefit for cosmetic products of avoiding the graying on black skins, resulting from the specific characteristics of the process of obtaining the product made up of lignin associated to the mixtures under the specific conditions of mixtures of ZnO and TiOz according to the present invention, said surprising result is confirmed, for example, by way of Figure 3, which presents a graph achieved in accordance with the standardization of a color space sphere.

As seen in Figure 3, in the comparative result of tones from the samples achieved usually without lignin and surprisingly with lignin under the conditions of associating mixtures of ZnO and of $TiO_2$ of the present invention, the base with lignin truly confers a subtle yet differentiated and perceptible darkening of the color with reddening, and the parameter that undergoes the biggest impact is the L, referring to the darkening of the color, which, according to a person skilled in the art, it could be said that according to the present invention "the sample is more somber".

Therefore, the composition presented by the present invention comprises a lilac composition formed by the solubilization of the lignin compound with pre-dispersions of physical filters such as ZnO and $TiO_2$, unexpectedly helping to maintain the tones of cosmetic bases with coloring for black skins.

Not only is there an unexpected effect of tone and booster effect of protection against blue light achieved by the lignin under the conditions of associating mixtures of ZnO and of $TiO_2$, but the present invention also adds the antioxidant potential to the resulting product, as vouched by assays using the DPPH technique.

In the DPPH assay, a solution at 10% of nanostructured lignin (2.5g in a 25mL volumetric flask) is prepared using ethanol. After extraction (30 minutes in an ultrasonic bath), the sample is submitted to centrifuging for 10 min, 3000 rpm for precipitation of particulate and filtered. In this assay, serial dilutions in ethanol are carried out so that said assay is conducted in the concentrations 0.5%; 0.75%; 1.0%; 1.5% and 2.0%, the analyses being performed in triplicate. The 2.5 ml of each sample solution, receives 1.0 mL of the DPPH solution (0.3 mM), and negative controls are also prepared (ethanol and DPPH) and one blank for each concentration (sample and ethanol). The reaction with DPPH is performed at room temperature (23°C ± 2.0) for 30 minutes.

After the reaction time, samples are evaluated in spectrophotometer at 518 nm, the analyses performed in accordance with the procedure DS.MET.000070 - Antioxidant Activity - Determination of EC50.

In analyzing the results, the percentage of antioxidant activity is considered by DPPH in each concentration being expressed by AA% calculated by the following formula:

$$AA\% = 100 - \frac{(\text{Abs from the sample} - \text{Abs from the blank}) \times 100}{\text{Abs from the negative control}}$$

It has to be pointed out that among the numerous mechanisms involved in the complex phenomenon of ageing, the oxidative process is cited as one of the most important. With ageing, a greater number of oxygen-reactive species is released in our skin, inducing a cascade of harmful oxidant reactions that are toxic to the skin. The toxic action of the free radicals on the skin can be reduced by the action of antioxidant substances added to the cosmetic formulations. Therefore, this detoxifying action can be measured by the antioxidant capacity of the formulation.

DPPH (1,1-diphenyl-2-picrylhydrazyl) is a free radical stable at room temperature that produces a violet methanolic solution. It is reduced in the presence of an antioxidant molecule (Hydrogen donor), resulting in a discolored methanolic solution. The assay measures the loss in the absorption when the DPPH radical is reduced by an antioxidant or free radical species, an assay being extremely useful in identifying the sample's potential to combat free radicals present in the environment.

The assay for the nanostructured lignin presents a linear calibration curve in the concentrations of 0.5%; 0.75%; 1.0%, with a correlation index ($R^2$) of 0.9974.

In turn, the value of EC50 is calculated based on the equation of this straight line, and the sample evaluated shows 50% of antioxidant activity at a concentration of 7.59 mg/mL, that is, in 0.76% of sample, as can be seen in Figure 4, which presents a graph of the antioxidant activity by DPPH based on the nanostructured lignin sample concentration.

Therefore, the analysis carried out indicates the antioxidant power of the sample evaluated, and the result of the DPPH analysis shows that based on a concentration of approximately 0.76%, the nanostructured lignin is capable of reducing by 50% the oxidation of the DPPH radical. Accordingly, products containing 0.76% of the raw material nanostructured lignin are capable of protecting the skin from external oxidizing agents.

Therefore, the cosmetic product resulting from the lignin processing under the conditions described above, with the addition of the specific mixtures of a suspension of ZnO and also of a dispersion composition of $TiO_2$, correlates the ideal lilac tone obtained in the final mixture, avoiding the graying on darker tone skins, due to the use of said cosmetic having an unexpected technical effect. Additionally, it also provides an improved effect of protection as booster protection against blue light. Lastly, as the product may also be comprised of at least 0.76% of raw material of nanostructured lignin, associated to the mixtures of a suspension of ZnO and to the mixtures of a dispersion composition of $TiO_2$, the present invention further confers protection to the user's skin against oxidizing agents.

**Claims**

1. A method for obtaining a lignin composition associated with ZnO and TiOz for a cosmetic product with coloring for black skin, to boost blue light protection and to protect the skin against external oxidizing agents, **characterized by** comprising the steps of:

   adding a dry lignin of high molecular weight to the composition of a product, said product being comprised of at least 0.5%, preferably at least 0.76% of raw material of nanostructured lignin, to the mixtures of a suspension of ZnO and to the mixtures of a dispersion composition of $TiO_2$;
   wherein the step of adding mixtures of a suspension of ZnO to the dry lignin of high molecular weight obtained, comprises adding 61.50 to 64.50 by weight % of Zinc Oxide, 31.00 to 35.00 by weight % of Butyloctyl Salicylate, 1.00 to 5.00 by weight % of Triethoxyaprilylsilane, and 1.00 to 2.50 by weight % of Alkyl Benzoate C12-C15; and
   wherein the step of adding mixtures of a dispersion composition of TiOz to the dry lignin of high molecular weight, comprises adding 0 to 60 by weight % of Dodecamethylpentasiloxane, 20 to 30 by weight % of Titanium Dioxide, and 5 to 10 by weight % of organo polysiloxane-modified polyglycerin.

2. The method for obtaining a lignin composition associated with ZnO and $TiO_2$ for a cosmetic product with coloring for black skin, to boost blue light protection and to protect the skin against external oxidizing agents, according to claim 1, wherein the step of adding a dispersion composition $TiO_2$ to the lignin is **characterized by** comprising the steps of adding particles of $TiO_2$ sized up to 10 nm in width and up to 50 nm in length.

3. The cosmetic product with coloring for black skin, to boost blue light protection and to protect the skin against external oxidizing agents, **characterized by** being obtained according to any one of the preceding claims, comprising dry lignin of high molecular weight associated with ZnO and $TiO_2$, of which at least 0.5%, preferably 0.76% of the raw material of said product, is made up of said nanostructured lignin.

## INTERNATIONAL SEARCH REPORT

| International application No. |
| --- |
| PCT/BR2021/050574 |

**A. CLASSIFICATION OF SUBJECT MATTER**

IPC: A61K8/27 (2006.01), A61K8/29 (2006.01), C07G1/00 (2011.01), A61Q17/04 (2006.01)
CPC: A61K8/27, A61K8/29, C07G1/00, A61Q17/04

According to International Patent Classification (IPC) or to both national classification and IPC

**B. FIELDS SEARCHED**

Minimum documentation searched (classification system followed by classification symbols)

A61K8/27, A61K8/29, C07G1/00, A61Q17/04,

Documentation searched other than minimum documentation to the extent that such documents are included in the fields searched

Base de patentes INPI - BR

Electronic data base consulted during the international search (name of data base and, where practicable, search terms used)

Derwent e Google Patents

**C. DOCUMENTS CONSIDERED TO BE RELEVANT**

| Category* | Citation of document, with indication, where appropriate, of the relevant passages | Relevant to claim No. |
| --- | --- | --- |
| Y | EP 3383526 B1 ( UNIV ORLEANS [FR]) 08 January 2020 (2020-01-08) (paragraphs 1 and 18; claim 1 and its dependents) | 1 to 3 |
| Y | Widsten, P. Lignin-Based Sunscreens - State-of-the-Art, Prospects and Challenges. Cosmetics, v. 7, n. 85, November 2020 (pages2,3 and 4, itens 2,3 and 4) | 1 to 3 |
| Y | US 6716418 B2 (AMCOL INTERNATIONAL CORP [US]) 06 April 2004 (2004-04-06) (abstract and claim 1) | 1 to 3 |
| A | WO 2017197530 A1 ( MORSELLA MICHELA [IT]) 23 November 2017 (2017-11-23) | 1 to 3 |

☒ Further documents are listed in the continuation of Box C.     ☒ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 01/04/2022 | 05/04/2022 |

| Name and mailing address of the ISA/ INSTITUTO NACIONAL DA PROPRIEDADE INDUSTRIAL Rua Mayrink Veiga n° 9, 8° andar cep: 20090-910. Centro - Rio de Janeiro/RJ | Authorized officer |
| --- | --- |
| Facsimile No. | Telephone No. |

Form PCT/ISA/210 (second sheet) (January 2015)

INTERNATIONAL SEARCH REPORT
Information on patent family members

International application No.

PCT/BR2021/050574

| EP 3383526 B1 | 2020-01-08 | EP 3383526 A1 | 2018-10-10 |
| | | FR 3044239 A1 | 2017-06-02 |
| | | JP 2018536668 A | 2018-12-13 |
| | | JP 6904951 B2 | 2021-07-21 |
| | | US 2018353395 A1 | 2018-12-13 |
| | | WO 2017093184 A1 | 2017-06-08 |
| US 6716418 B2 | 2004-04-06 | US 2003152531 A1 | 2003-08-14 |
| | | AT 367790 T | 2007-08-15 |
| | | AU 2002243838 A1 | 2002-08-19 |
| | | CA 2437743 A1 | 2002-08-15 |
| | | DE 60221354 D1 | 2007-09-06 |
| | | EP 1357892 A1 | 2003-11-05 |
| | | JP 2004522751 A | 2004-07-29 |
| | | JP 4137639 B2 | 2008-08-20 |
| | | KR 20040025893 A | 2004-03-26 |
| | | MX PA03006945 A | 2004-01-29 |
| | | MX 253435 B | 2008-01-16 |
| | | PL 364144 A1 | 2004-12-13 |
| | | US 2002182155 A1 | 2002-12-05 |
| | | US 6500411 B2 | 2002-12-31 |
| | | WO 02062310 A1 | 2002-08-15 |
| WO 2017197530 A1 | 2017-11-23 | CA 3030443 A1 | 2017-11-23 |
| | | EP 3458026 A1 | 2019-03-27 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 106309212 A
- JP 2006052197 B
- US 6500411 B
- KR 20190109032
- KR 20190083182
- WO 2017197530 A
- WO 2005048970 A
- WO 2010093573 A
- WO 2017093184 A
- IN 201811048498
- JP 3751630 B
- WO 18004194 A

**Non-patent literature cited in the description**

- **JOSÉ MANUEL GUTIÉRREZ-HERNÁNDEZ et al.** Use of Agave Tequilana-Lignin and Zinc Oxide Nanoparticles for Skin Photoprotection. *Journal of photochemistry and photobiology. B, Biology,* October 2016